Europäisches Patentamt

European Patent Office

Office européen des brevets

(19)

(11) Veröffentlichungsnummer: **0 217 141**
**B1**

(12) **EUROPÄISCHE PATENTSCHRIFT**

(45) Veröffentlichungstag der Patentschrift: 26.09.90

(21) Anmeldenummer: 86111922.0

(22) Anmeldetag: 28.08.86

(51) Int. Cl.⁵: **A 61 K 31/57,** A 61 K 47/00,
A 61 K 9/10

(54) **Hydrocortison-17-propionat-21-acetat enthaltende W/O-Creme.**

(30) Priorität: 28.09.85 DE 3534742

(43) Veröffentlichungstag der Anmeldung:
08.04.87 Patentblatt 87/15

(45) Bekanntmachung des Hinweises auf die
Patenterteilung:
26.09.90 Patentblatt 90/39

(84) Benannte Vertragsstaaten:
AT BE CH DE FR GB IT LI LU NL SE

(56) Entgegenhaltungen:
EP-A-0 098 566
EP-A-0 131 821
DE-A-2 851 544
FR-A-2 539 991
FR-A-2 539 992

CHEMICAL ABSTRACTS, Band 103, 1985, Seite
358, Zusammenfassung Nr. 220828m,
Columbus, Ohio, US; & JP-A-60 156 608
(TAISHO PHARMACEUTICAL CO., LTD) 16-08-
1985

Die Akte enthält technische Angaben, die nach
dem Eingang der Anmeldung eingereicht
wurden und die nicht in dieser Patentschrift
enthalten sind.

(73) Patentinhaber: Beiersdorf Aktiengesellschaft
Unnastrasse 48
D-2000 Hamburg 20 (DE)

(72) Erfinder: Sattler, Henning, Dr.
Jahnring 15
D-2000 Hamburg 60 (DE)
Erfinder: Asmussen, Bodo, Dr.
Dorotheenweg 1a
D-2071 Ammersbek (DE)

(56) References cited:
Merck Index, 10. Auflage(1983), S.594

**Beschreibung**

Die vorliegende Erfindung bezieht sich auf eine neue Creme, die Hydrocortison-17-propionat-21-acetat enthält.

Aus der DE—OS—3 402 877 ist bereits eine ölige Salbe bzw. Fettsalbe mit dem Wirkstoff Hydrocortison-17-butyrat-21-propionat bekannt.

Salben, die keinen oder nur einen sehr geringen Wassergehalt aufweisen, bilden keine Emulsionen und sind somit keine Cremes. Hinsichtlich der Wirkstoffresorption sind solche Zubereitungen nicht immer befriedigend. Auch ist ihre Anwendung mit Unannehmlichkeiten verbunden.

Weiterhin ist aus der DE—OS—34 02 880 eine O/W-Creme mit dem Wirkstoff Hydrocortison-17-butyrat-21-propionat bekannt.

Die beschriebene Cremegrundlage hat sich jedoch nicht immer, insbesondere hinsichtlich der Lagerungsstabilität, beziehungsweise der Beständigkeit des Wirkstoffgehalts, als zufriedenstellend erwiesen. Dies ist in besonderem Maße dann der Fall, wenn andere Hydrocortisondiester als der dort beschriebene Diester mit der angegebenen Grundlage verwendet werden.

Aufgabe der Erfindung ist es, eine Hydrocortison-17-propionat-21-acetat enthaltende W/O-Creme bereitzustellen, die eine gute Lagerungsstabilität und eine hohe Absorption des Wirkstoffes durch die Haut gewährleistet.

Diese Aufgabe wird gelöst durch eine W/O-Creme, die dadurch gekennzeichnet ist, daß sie

0,01—0,5 Gew.% Hydrocortison-17-propionat-21-acetat,
1—10 Gew.% Bienenwachs oder Bienenwachssubstitut,
1—25 Gew.% flüssiges Paraffin,
25—75 Gew.% weißes Vaselin,
0—2 Gew.% Aluminiumstearat,
2—20 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl,
0—2 Gew.% wasserhaltiges Magnesiumsulfat und
10—60 Gew.% Wasser

enthält.

Vorzugsweise enthält die W/O-Creme:

0,025—0,2 Gew.% Hydrocortison-17-propionat-21-acetat
2—8 Gew.% Bienenwachs oder Bienenwachssubstitut
5—15 Gew.% flüssiges Paraffin
40—60 Gew.% weißes Vaselin
0,2—1 Gew.% Aluminiumstearat
5—15 Gew.% W/O-Emulgator auf der Basis von geradkettigen und verzweigten Kohlenwasserstoffen, Glyceryloleat und Wollwachsalkoholen oder Polyglyceryl-(4-isostearat sowie hydriertem Rizinusöl
0,5—1 Gew.% wasserhaltiges Magnesiumsulfat und
15—40 Gew.% Wasser,

insbesondere aber:

0,13 Gew.% Hydrocortisondiester
5 Gew.% Bienenwachs oder Bienenwachsubstitut
9 Gew.% flüssiges Paraffin
50,5 Gew.% weißes Vaselin
0,5 Gew.% Aluminiumstearat
10 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl,
0,7 Gew.% wasserhaltiges Magnesiumsulfat und
24,17 Gew.% Wasser.

Die erfindungsgemäßen Cremes zeichnen sich durch eine besonders hohe Lagerungsstabilität und gute Wirksamkeit aus. Auch nach mehrjähriger Lagerung ist praktisch keine Abnahme des Wirkstoffgehalts meßbar.

Bevorzugt werden Bienenwachssubstitute auf der Basis von Partialglyceriden und Estern langkettiger Fettsäuren die unter der Handelsbezeichnung "Cutina BW" (Firma Henkel) vertrieben werden.

- Als flüssiges Paraffin wird vorzugsweise dünnflüssiges Paraffin verwendet.

Vorzugsweise enthält die Creme Aluminiumstearat in der angegebenen Menge.

2

Besonders bevorzugt werden W/O-Emulgatoren auf der Basis eines Gemisches von geradkettigen und verzweigten Kohlenwasserstoffen, Glyceryloleat und Wollwachsalkoholen oder Polyglyceryl-(4)-isostearat und hydriertem Rizinusöl, deren Kohlenwasserstoffanteil vorzugsweise von Paraffin, Vaselin und Ozokerit gebildet wird.

Solche Emulgatoren sind in der Literatur beschrieben (CTFA) Cosmetic Ingredient Dictionary, dritte Ausgabe, The Cosmetic, Toiletry and Fragrance Association, Inc. 1110 Vermont Avenue, N.W. Washington, D.C. 20005, insbesondere S. 505, 230, 196, 123, 111, 112, 243) und unter den Handelsnamen Protegin, Protegin W, Protegin WX und Protegin X erhältlich (Firma Goldschmidt, DE). Bevorzugt werden die Typen Protegin W und WX, insbesondere aber Protegin WX, das sich als besonders vorteilhaft erwiesen hat. Protegin und Protegin X enthalten Wollwachsalkohole, Protegin W und Protegin WX dagegen Polyglyceryl-(4)-isostearat und hydriertes Rizinusöl.

Der Kohlenwasserstoffanteil von Protegin und Protegin X besteht aus Paraffin, Vaselin und Ozokerit, der von Protegin W und Protegin WX aus Vaselin und Ozokerit.

Vorzugsweise enthält die erfindungsgemäße Creme Magnesiumsulfat, insbesondere wird Magnesiumsulfat .7H$_2$O eingesetzt.

Weiterhin können Zusatzstoffe wie Glycerin, Propylenglykol, Isopropylfettsäureester wie Isopropylmyristat und Isopropylpalmitat, Wachse, beispielsweise Kohlenwasserstoffwachse wie Ozokerit sowie Bienenwachs und Walrat und deren Substitute in kleineren Anteilen enthalten sein, ebenso wie Mittel zur pH-Regulierung und Konservierungsmittel, die aber im allgemeinen in der erfindungsgemäßen Creme nicht erforderlich sind.

Mit der neuen erfindungsgemäßen Creme wird eine Hydrocortison-17-propionat-21-acetat, enthaltende W/O-Creme geschaffen, die sich durch gute Wirksamkeit und hohe Lagerstabilität auszeichnet.

Zur Herstellung der Creme werden die Bestandteile der Fettphase wie Vaselin, Paraffin, Bienenwachs oder Bienenwachssubstitut, Aluminiumstearat und der Emulgator geschmolzen und auf 60° bis 80°C gebracht. Das Magnesiumsulfat wird in Wasser gelöst, wobei die wäßrige Phase ebenfalls auf 60—80°C, erwärmt wird. Beide Phasen werden gemischt und bei einer Temperatur von etwa 60°C wird der Wirkstoff Hydrocortisondiester zugegeben. Unter Rühren läßt man die Masse abkühlen und erstarren.

Aus der erfindungsgemäßen Creme, die eine hervorragende Lagerungsstabilität aufweist, wird der Wirkstoff sehr gut absorbiert. Sie wird zur Behandlung von Ekzemen, Dermatitis, Psoriasis sowie Entzündungen verwendet.

Zur Heilung oder Behandlung dieser Erkrankungen kann die erfindungsgemäße Creme topisch auf die geschädigten Stellen aufgebracht werden. Die aufgebrachte Menge der Creme variiert entsprechend der Konzentration des Wirkstoffs der Creme. Im allgemeinen wird eine geeignete Menge auf die geschädigte Stelle mehrmals täglich je nach Schwere der zu behandelnden Erkrankung aufgebracht.

Die folgenden Beispiele dienen zur Erläuterung der Erfindung:

Beispiel 1

Mit den angegebenen Bestandteilen wird eine W/O-Creme hergestellt:

| | |
|---|---|
| Hydrocortison-21-acetat-17-propionat | 0,127 g |
| W/O-Emulgator auf der Basis von geradkettigen und verzweigten Kohlenwasserstoffen, Glyceryl-oleat und Polyglyceryl-(4)-isostearat sowie hydriertem Rizinusöl (Protegin WX) | 10 g |
| Bienenwachssubstitut (Cutina BW) | 5 g |
| flüssiges Paraffin, | 9 g |
| weißes Vaselin, | 50,5 g |
| Aluminiumstearat | 0,5 g |
| Magnesiumsulfat | 0,7 g |
| gereinigtes Wasser | 24,173 g |
| | 100,000 g |

Bienenwachssubstitut, Vaselin, Paraffin, Aluminiumstearat und der Emulgator werden auf 75°C erhitzt, Magnesiumsulfat und Wasser werden unter Auflösung des Salzes ebenfalls auf 75°C erhitzt. Die Phasen werden gemischt, nach Abkühlen auf 60°C wird das Hydrocortison-17-propionat-21-acetat zugegeben und die Masse bis zu Erkalten gerührt.

Biespiel 2
Mit den angegebenen Bestandteile wird eine W/O-Creme hergestellt:

| | |
|---|---|
| Hydrocortison-21-acetat-17-propionat | 0,050 g |
| W/O-Emulgator auf der Basis von geradkettigen und verzweigten Kohlenwasserstoffen, Glyceryloleat und Polyglyceryl-(4)-isostearat sowie hydriertem Rizinusöl (Protegin WX) | 5,0 g |
| Bienenwachssubstitut (Cutina BW) | 6,0 g |
| flüssiges Paraffin, | 15,0 g |
| weißes Vaselin, | 50,0 g |
| gereinigtes Wasser | 23,95 g |
| | 100,000 g |

Bienenwachssubstitut, Vaselin, Paraffin und der Emulgator werden auf 75°C erhitzt. Das wasser wird auf 75°C erhitzt. Die Phasen werden gemischt, nach Abkühlen auf 60°C wird das Hydrocortison-17-propionat-21-acetat zugegeben und die Masse bis zum Erkalten gerührt.

Beispiel 3
Mit den angegebenen Bestandteilen wird eine W/O-Creme hergestellt:

| | |
|---|---|
| Hydrocortison-21-acetat-17-propionat | 0,100 g |
| W/O-Emulgator auf der Basis von geradkettigen und verzweigten Kohlenwasserstoffen, Glyceryloleat und Polyglyceryl-(4)-isostearat sowie hydriertem Rizinusöl (Protegin W) | 15,0 g |
| Bienenwachssubstitut (Cutina BW) | 5,0 g |
| flüssiges Paraffin, | 14,0 g |
| weißes Vaselin, | 41,0 g |
| Magnesiumsulfat | 0,5 g |
| gereinigtes Wasser | 24,4 g |
| | 100,000 g |

Bienenwachssubstitut, Vaselin, Paraffin, und der Emulgator werden auf 75°C erhitzt. Magnesiumsulfat und Wasser werden unter Auflösung des Salzes ebenfalls auf 75°C erhitzt. Die Phasen werden gemischt, nach Abkühlen auf 60°C wird das Hydrocortison-17-propionat-21-acetat zugegeben und die Masse bis zum Erkalten gerührt.

**Patentansprüche für die Vertragsstaaten: BE, CH, DE, FR, GB, IT, LI, LU, NL, SE**

1. Hydrocortisondiester enthaltende Creme, dadurch gekennzeichnet, daß sie

0,01—0,5 Gew.% Hydrocortison-17-propionat-21-acetat
1—10 Gew.% Bienenwachs oder Bienenwachssubstitut,
1—25 Gew.% flüssiges Paraffin,
25—75 Gew.% weißes Vaselin
0—2 Gew.% Aluminiumstearat,
2—20 Gew.% W/O-Emulgator auf der Basis von geradkettigen und verzweigten Kohlenwasserstoffen, Glyceryloleat und Wollwachsalkohlen oder Polyglyceryl-(4)-isostearat sowie hydriertem Rizinusöl,
0—2 Gew.% wasserhaltiges Magnesiumsulfat und
10—60 Gew.% Wasser enthält.
2. Creme gemäß Anspruch 1, dadurch gekennzeichnet, daß sie

0,13 Gew.% Hydrocortison-17-propionat-21-acetat,
5 Gew.% Bienenwachs oder Bienenwachssubstitut
9 Gew.% flüssiges Paraffin
50,5 Gew.% weißes Vaselin
0,5 Gew.% Aluminiumstearat

10 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl,

0,7 Gew.% wasserhaltiges Magnesiumsulfat und

24,17 Gew.% Wasser enthält.

3. Creme gemäß Anspruch 1, zur Behandlung von Entzündungen, Psoriasis und Ekzemen.

4. Verfahren zur Herstellung der Creme gemäß Anspruch 1, dadurch gekennzeichnet, daß man

1—10 Gew.% Bienenwachs oder Bienenwachssubstitut,

1—25 Gew.% flüssiges Paraffin,

25—75 Gew.% weißes Vaselin,

0—2 Gew.% Aluminiumstearat,

2—20 Gew.% W/O-Emulgator auf der Basis von geradkettigen und verzweigten Kohlenwasserstoffen, Glyceryloleat und Wollwachsalkohlen oder Polyglyceryl-(4)-isostearat sowie hydriertem Rizinusöl, auf 60—80°C bringt, mit

0—2 Gew.% wasserhaltigem Magnesiumsulfat gelöst in

10—60 Gew.% Wasser von 60—80°C mischt, bei etwa 60°C

0,01—0,5 Gew.% Hydrocortison-17-propionat-21-acetat zugibt und unter Rühren die Masse erstarren laßt.


**Patentansprüche für der Vertragsstaat: AT**

1. Hydrocortisondiester enthaltenden Creme, dadurch gekennzeichnet, daß man

0,01—0,5 Gew.% Hydrocortison-17-propionat-21-acetat

1—10 Gew.% Bienenwachs oder Bienenwachssubstitut,

1—25 Gew.% flüssiges Paraffin,

25—75 Gew.% weißes Vaselin

0—2 Gew.% Aluminiumstearat,

2—20 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl,

0—2 Gew.% wasserhaltiges Magnesiumsulfat und

10—60 Gew.% Wasser zu einer Creme verarbeitet.

2. Verfahren zur Herstellung einer Creme gemäß Anspruch 1, dadurch gekennzeichnet, daß man

0,13 Gew.% Hydrocortison-17-propionat-21-acetat,

5 Gew.% Bienenwachs oder Bienenwachssubstitut,

9 Gew.% flüssiges Paraffin

50,5 Gew.% weißes Vaselin

0,5 Gew.% Aluminiumstearat

10 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl,

0,7 Gew.% wasserhaltiges Magnesiumsulfat und

24,17 Gew.% Wasser zu einer Creme verarbeitet.

3. Verfahren zur Herstellung einer Creme zur Behandlung von Entzündungen, Psoriasis und Ekzemen gemäß Anspruch 1.

4. Verfahren zur Herstellung der Creme gemäß Anspruch 1, dadurch gekennzeichnet, daß man

1—10 Gew.% Bienenwachs oder Bienenwachssubstitut,

1—25 Gew.% flüssiges Paraffin,

25—75 Gew.% weißes Vaselin,

0—2 Gew.% Aluminiumstearat,

2—20 Gew.% W/O-Emulgator auf der Basis von geradkettigen und verzweigten Kohlenwasserstoffen, Glyceryloleat und Wollwachsalkoholen oder Polyglyceryl-(4)-isostearat sowie hydriertem Rizinusöl, auf 60—80°C bringt, mit

0—2 Gew.% wasserhaltigem Magnesiumsulfat gelöst in

5

10—60 Gew.% Wasser von 60—80°C mischt, bei etwa 60°C
0,01—0,5 Gew.-% Hydrocortison-17-propionat-21-acetat zugibt und unter Rühren die Masse erstarren läßt.

**Revendications pour les Etats Contractants: BE, CH, DE, FR, GB, IT, LI, LU, NL, SE**

1. Crème contenant un diester d'hydrocortisone, caractérisée en ce qu'elle contient

0,01—0,5% en poids de 17-propionate 21-acétate d'hydrocortisone,
1—10% en poids de cire d'abeille ou de succédané de cire d'abeille,
1—25% en poids de paraffine liquide,
25—75% en poids de vaseline blanche,
0—2% en poids de stéarate d'aluminium,
2—20% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogénée,
0—2% en poids de sulfate de magnésium hydraté, et
10—60% en poids d'eau.

2. Crème selon la revendication 1, caractérisée en ce qu'elle contient

0,13% en poids de 17-propionate 21-acétate d'hydrocortisone,
5% en poids de cire d'abeille ou de succédané de cire d'abeille,
9% en poids de paraffin liquide,
50,5% en poids de vaseline blanche,
0,5% en poids de stéarate d'aluminium,
10% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogénée,
0,7% en poids de sulfate de magnésium hydraté, et
24,17% en poids d'eau.

3. Crème selon la revendication 1, pour le traitement d'inflammations, de psoriasis et d'eczémas.
4. Procédé de préparation de la crème selon la revendication 1, caractérisé en ce que l'on porte à 60—80°C

1—10% en poids de cire d'abeille ou de succédané de cire d'abeille,
1—25% en poids de paraffine liquide,
25—75% en poids de vaseline blanche,
0—2% en poids de stéarate d'aluminium,
2—20% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogénée, que l'on mélange avec
0—2% en poids de sulfate de magnésium hydraté, dissous dans
10—60% en poids d'eau à 60—80°C, et que l'on ajoute, à environ 60°C,
0,01—0,5% en poids de 17-propionate 21-acétate d'hydrocortisone, et que l'on laisse la composition de solidifier sous agitation.

**Revendications pour l'Etat Contractant: AT**

1. Procédé de préparation d'une crème contenant un diester d'hydrocortisone, caractérisé en ce que l'on traite, pour obtenir une crème,

0,01—0,5% en poids de 17-propionate 21-acétate d'hydrocortisone,
1—10% en poids de cire d'abeille ou de succédané de cire d'abeille,
1—25% en poids de paraffine liquide,
25—75% en poids de vaseline blanche,
0—2% en poids de stéarate d'aluminium,
2—20% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogénée,
0—2% en poids de sulfate de magnésium hydraté, et
10—60% en poids d'eau.

2. Procédé de préparation d'une crème selon la revendication 1, caractérisé en ce que l'on traite, pour obtenir une crème,

0,13% en poids de 17-propionate 21-acétate d'hydrocortisone,
5% en poids de cire d'abeille ou de succédané de cire d'abeille,
9% en poids de paraffine liquide,
50,5% en poids de vaseline blanche,
0,5% en poids de stéarate d'aluminium,
10% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogénée,
0,7% en poids de sulfate de magnésium hydraté, et
24,17% en poids d'eau.

3. Procédé de préparation d'une crème pour le traitement d'inflammations, de psoriasis et d'eczémas selon la revendication 1.

4. Procédé de préparation de la crème selon la revendication 1, caractérisé en ce que l'on porte à 60—80°C

1—10% en poids de cire d'abeille ou de succédané de cire d'abeille,
1—25% en poids de paraffine liquide,
25—75% en poids de vaseline blanche,
0—2% en poids de stéarate d'aluminium,
2—20% en poids d'émulsifiant eau dans l'huile à base d'hydrocarbures à chaîne droite et ramifiée, d'oléate de glycéryle et d'alcools de cire de lanoline ou d'isostéarate de polyglycéryle-(4) et d'huile de ricin hydrogéne, que l'on mélange avec
0—2% en poids de sulfate de magnésium hydraté, dissous dans
10—60% en poids d'eau à 60—80°C, et que l'on ajoute, à environ 60°C,
0,01—0,5% en poids de 17-propionate 21-acétate d'hydrocortisone, et que l'on laisse la composition se solidifier sous agitation.

**Claims for the Contracting States: BE, CH, DE, FR, GB, IT, LI, LU, NL, SE**

1. Cream containing hydrocortisone diester, characterized in that it contains

0.01—0.5% by weight of hydrocortisone 17-propionate 21-acetate,
1—10% by weight of beeswax or beeswax substitute,
1—25% by weight of liquid paraffin,
25—75% by weight of white vaseline,
0—2% by weight of aluminium stearate,
2—20% by weight of W/O emulsifier based on straight-chain and branched hydrocarbons, glyceryl oleate and wool wax alcohols or polyglyceryl-(4) isostearate and hydrogenated castor oil,
0—2% by weight of hydrated magnesium sulphate and
10—60% by weight of water.

2. Cream according to Claim 1, characterized in that it contains

0.13% by weight of hydrocortisone 17-propionate 21-acetate,
5% by weight of beeswax or beeswax substitute,
9% by weight of liquid paraffin,
50.5% by weight of white vaseline,
0.5% by weight of aluminium stearate,
10% by weight of W/O emulsifier based on straight-chain and branched hydrocarbons, glyceryl oleate and wool wax alcohols or polyglyceryl-(4) isostearate and hydrogenated castor oil,
0.7% by weight of hydrated magnesium sulphate and
24.17% by weight of water.

3. Cream according to Claim 1 for the treatment of inflammations, psoriasis and eczemas.

4. Process for the preparation of the cream according to Claim 1, characterized in that

1—10% by weight of beeswax or beeswax substitute,
1—25% by weight of liquid paraffin,
25—75% by weight of white vaseline,

0—2% by weight of aluminium stearate,

2—20% by weight of W/O emulsifier based on straight-chain and branched hydrocarbons, glyceryl oleate and wool wax alcohols or polyglyceryl-(4) isostearate and hydrogenated castor oil are brought to 60—80°C and mixed with

0—2% by weight of hydrated magnesium sulphate dissolved in

10—60% by weight of water at 60—80°C, and, at about 60°C,

0.01—0.5% by weight of hydrocortisone 17-propionate 21-acetate is added, and the composition is allowed to solidify while stirring.

## Claims for the Contracting State: AT

1. Process for the preparation of a cream containing hydrocortisone diester, characterized in that

0.01—0.5% by weight of hydrocortisone 17-propionate 21-acetate,

1—10% by weight of beeswax or beeswax substitute,

1—25% by weight of liquid paraffin,

25—75% by weight of white vaseline,

0—2% by weight of aluminium stearate,

2—20% by weight of W/O emulsifier based on straight-chain and branched hydrocarbons, glyceryl oleate and wool wax alcohols or polyglyceryl-(4) isostearate and hydrogenated castor oil,

0—2% by weight of hydrated magnesium sulphate and

10—60% by weight of water are processed to give a cream.

2. Process for the preparation of a cream according to Claim 1, characterized in that

0.13% by weight of hydrocortisone 17-propionate 21-acetate,

5% by weight of beeswax or beeswax substitute,

9% by weight of liquid paraffin,

50.5% by weight of white vaseline,

0.5% by weight of aluminium stearate,

10% by weight of W/O emulsifier based on straight-chain and branched hydrocarbons, glyceryl oleate and wool wax alcohols or polyglyceryl-(4) isostearate and hydrogenated castor oil, 0.7% by weight of hydrated magnesium sulphate and

24.17% by weight of water are processed to give a cream.

3. Process for the preparation of a cream for the treatment of inflammations, psoriasis and eczemas according to Claim 1.

4. Process for the preparation of the cream according to Claim 1, characterized in that

1—10% by weight of beeswax or beeswax substitute,

1—25% by weight of liquid paraffin,

25—75% by weight of white vaseline,

0—2% by weight of aluminium stearate,

2—20% by weight of W/O emulsifier based on straight-chain and branched hydrocarbons, glyceryl oleate and wool wax alcohols or polyglyceryl-(4) isostearate and hydrogenated castor oil are brought to 60—80°C and mixed with

0—2% by weight of hydrated magnesium sulphate dissolved in

10—60% by weight of water at 60—80%, and, at about 60°C,

0.01—0.5% by weight of hydrocortisone 17-propionate 21-acetate is added, and the composition is allowed to solidify while stirring.